# EUROPEAN PATENT APPLICATION

(11) **EP 3 607 885 A1**
(43) Date of publication of application: **12.02.2020**
(21) Application number: 19191104.9
(22) Date of filing: 09.08.2019
(51) Int. Cl.: A61B 8/00

(54) **ULTRASONIC DIAGNOSTIC APPARATUS**

(30) Priority: 09.08.2018 KR 20180093409
(71) Applicant: Samsung Medison Co., Ltd., Hongcheon-gun, Gangwon-do, 25108 (KR)
(72) Inventor: HYUN, Dong Gyu, Gyeonggi-do (KR); KIM, Hyoung Jin, Seoul (KR); JUN, Dong Soo, Seoul (KR)
(74) Representative: Jacobs, Bart

(57) **Abstract**

Disclosed is an ultrasonic diagnostic apparatus capable of easily obtaining an ultrasonic image for the same position of the same target object at different points of time. The ultrasonic diagnostic apparatus includes a probe configured to obtain ultrasonic data for an arbitrary position inside a target object, an image generator to generate an ultrasonic image for the arbitrary position using the ultrasonic data, a reference member provided to be in contact with the target object to set a reference position in the target object, a marker disposed on one side of the reference member, a sensor disposed on one side of the probe and configured to obtain a positional relationship with the marker, and a position calculator configured to calculate a positional relationship between the reference position and the arbitrary position using the positional relationship between the marker and the sensor.

## Description

### BACKGROUND

### 1. Technical Field

The disclosure relates to an ultrasonic diagnostic apparatus, and more particularly, to an ultrasonic diagnostic apparatus capable of easily obtaining an ultrasonic image for the same site of the same target object at different points of time.

### 2. Description of the Related Art

An ultrasonic diagnostic apparatus may obtain an image for a site inside a target object by radiating an ultrasonic signal generated from a transducer of a probe to the target object and receiving the information of an ultrasonic echo signal reflected from the target object. In particular, the ultrasonic diagnostic apparatus may be used for medical purposes such as observation of the inside of a target object, detection of a foreign object, and measurement of an injury. Such an ultrasonic diagnostic apparatus is advantageous in that it has high stability, may display images in real time, and is safe because there is no radiation exposure, compared with a diagnostic apparatus using an X-ray, so that the ultrasonic diagnostic apparatus is widely used together with other image diagnostic apparatuses.

In the case of rehabilitation and chronic illness patients, continuous observation of treatment and progress is required. In this way, when an ultrasonic image for the same affected part of the same patient is required repeatedly with a time difference, it is very cumbersome to repeatedly find the precise location of the affected part that was monitored on a previous visit.

### SUMMARY

It is an aspect of the disclosure to provide an ultrasonic diagnostic apparatus capable of easily obtaining an ultrasonic image for the same position of the same target object at different points of time.

It is another aspect of the disclosure to provide an ultrasonic diagnostic apparatus capable of setting a reference position to a target object using a reference member and identifying a position of a probe with respect to the reference position.

Additional aspects of the disclosure will be set forth in part in the description which follows and, in part, will be obvious from the description, or may be learned by practice of the disclosure.

In accordance with an aspect of the disclosure, an ultrasonic diagnostic apparatus includes a probe configured to obtain ultrasonic data for an arbitrary position inside a target object, an image generator to generate an ultrasonic image for the arbitrary position using the ultrasonic data, a reference member provided to be in contact with the target object to set a reference position in the target object, a marker disposed on one side of the reference member, a sensor disposed on one side of the probe and configured to obtain a positional relationship with the marker, and a position calculator configured to calculate a positional relationship between the reference position and the arbitrary position using the positional relationship between the marker and the sensor.

The ultrasonic diagnostic apparatus may further include a display to display the ultrasonic image and the positional relationship.

The reference member may include a reference setting part provided to be in contact with the target object, and the reference member may set one point of the target object in contact with the reference setting part as the reference position.

The position calculator may include a first position calculator to obtain a positional relationship between the reference position and the marker, and a second position calculator to obtain a positional relationship between the sensor and the arbitrary position.

When the probe obtains ultrasonic data for the target object at a first point of time and a second point of time different from the first point of time, the display may display an ultrasonic image for a first position obtained at the first point of time and an ultrasonic image for the first position obtained at the second point of time.

The ultrasonic diagnostic apparatus may further include a controller, when the probe obtains ultrasonic data for the target object at a first point of time and a second point of time different from the first point of time, to determine whether or not a first position of the probe at the first point of time and a second position of the probe at the second point of time correspond to each other.

The display, when the first position and the second position correspond to each other, may display an ultrasonic image for the first position obtained at the first point of time and an ultrasonic image for the second position obtained at the second point of time.

The ultrasonic diagnostic apparatus may further include a controller, when the probe obtains ultrasonic data for a second position of the target object at a second point of time different from a first point of time after obtaining ultrasonic data for a first position of the target object at the first point of time, to determine whether or not the first position and the second position correspond to each other.

The controller, when it is determined that the first position and the second position do not correspond to each other, may select a movement route for the probe to move to the first position, and the display displays the movement route from the position of the probe to the first position.

In accordance with another aspect of the disclosure, an ultrasonic diagnostic apparatus includes a probe configured to obtain ultrasonic data for an arbitrary position inside a target object, an image generator to generate an ultrasonic image for the arbitrary position using the ultrasonic data, a maker disposed on one side of the probe, a reference member provided to be in contact with a portion of the target object to set a reference position in the target object, a sensor fixed to one side of the reference member and configured to obtain a positional relationship with the marker, and a position calculator configured to calculate a positional relationship between the reference position and the arbitrary position using the positional relationship between the marker and the sensor.

The ultrasonic diagnostic apparatus may further include a display to display the ultrasonic image and the positional relationship.

The reference member may include a reference setting part provided to be in contact with the target object, and the reference member may set one point of the target object in contact with the reference setting part as the reference position.

The position calculator may include a first position calculator to obtain a positional relationship between the reference position and the marker, and a second position calculator to obtain a positional relationship between the sensor and the arbitrary position.

### BRIEF DESCRIPTION OF THE DRAWINGS

These and/or other aspects of the disclosure will become apparent and more readily appreciated from the following description of the embodiments, taken in conjunction with the accompanying drawings of which:
FIG. 1 is a view illustrating an ultrasonic diagnostic apparatus according to an embodiment of the disclosure;
FIG. 2 is a view illustrating a reference member, a target object, and a probe in the ultrasonic diagnostic apparatus according to an embodiment of the disclosure;
FIG. 3 is a block diagram illustrating the configuration of the ultrasonic diagnostic apparatus according to an embodiment of the disclosure;
FIG. 4 is a block diagram illustrating the configuration of a display unit in the ultrasonic diagnostic apparatus according to an embodiment of the disclosure;
FIG. 5 is a view illustrating a reference member, a target object, and a probe in an ultrasonic diagnostic apparatus according to another embodiment of the disclosure;
FIG. 6 is a view illustrating a reference member, a target object, and a probe in an ultrasonic diagnostic apparatus according to another embodiment of the disclosure; and
FIG. 7 is a view illustrating a reference member, a target object, and a probe in an ultrasonic diagnostic apparatus according to another embodiment of the disclosure.

### DETAILED DESCRIPTION

The embodiments described in the present specification and the configurations shown in the drawings are only examples of preferred embodiments of the disclosure, and various modifications may be made at the time of filing of the disclosure to replace the embodiments and drawings of the present specification.

Like reference numerals or signs in the respective drawings of the present specification represent parts or components that perform substantially the same functions.

The terms used in the present specification are for the purpose of describing the embodiments and are not intended to restrict and/or to limit the disclosure. The singular expressions herein may include plural expressions, unless the context clearly dictates otherwise. Also, the terms "comprises" and "has" are intended to indicate that there are features, numbers, steps, operations, elements, parts, or combinations thereof described in the specification, and do not exclude the presence or addition of one or more other features, numbers, steps, operations, elements, parts, or combinations thereof.

It will be understood that, although the terms "first" and "second" may be used herein to describe various components, these components is not be limited by these terms and are only used to distinguish one component from another. For example, without departing from the scope of the disclosure, the first component may be referred to as a second component, and similarly, the second component may also be referred to as a first component.

The term "and/or" includes any combination of a plurality of related items or any one of a plurality of related items.

Hereinafter, an ultrasonic diagnostic apparatus according to embodiments of the disclosure will be described in detail with reference to the accompanying drawings.

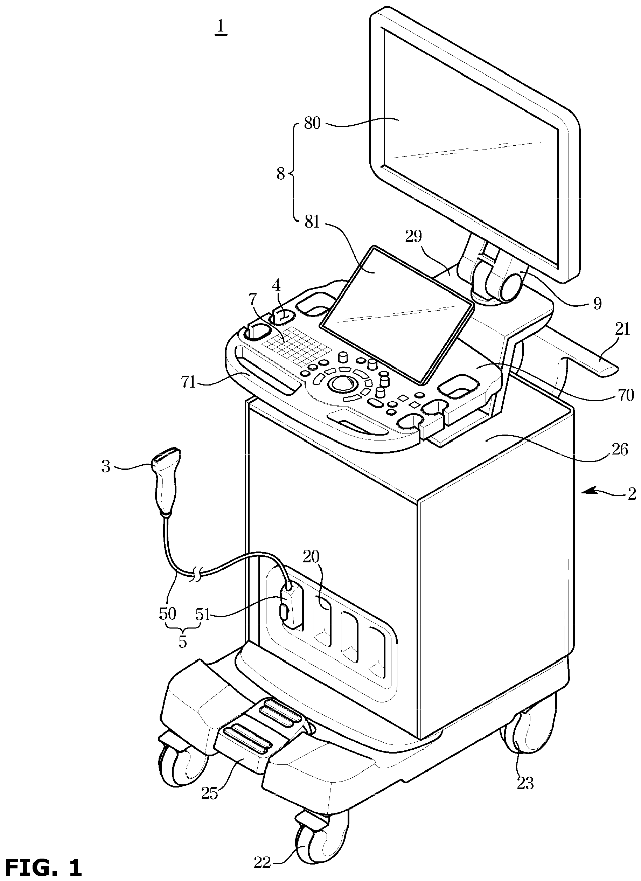

FIG. 1 is a perspective view illustrating an ultrasonic diagnostic apparatus according to an embodiment of the disclosure. Hereinafter, an ultrasonic diagnostic apparatus including a wired ultrasonic probe will be described as an example, but the disclosure is not limited thereto. Therefore, an ultrasonic diagnostic apparatus according to an embodiment of the disclosure may include a wireless ultrasonic probe.

Referring to FIG. 1, an ultrasonic diagnostic apparatus 1 according to an embodiment of the disclosure includes a main body 2, a probe 3, an input 7, and a display member 8. The display member 8 may include a main display 80 and an auxiliary display 81.

The display member 8 may display an ultrasonic image obtained in an ultrasonic diagnostic process. In addition, the display member 8 may display an application related to the operation of the ultrasonic diagnostic apparatus 1. For example, the main display 80 may display ultrasonic images obtained in an ultrasonic diagnostic process, and the auxiliary display 81 may display matters related to the operation of the ultrasonic diagnostic apparatus 1.

The main display 80 or the auxiliary display 81 may be implemented as a cathode ray tube (CRT) or a liquid crystal display (LCD). The main display 80 or the auxiliary display 81 may be coupled to the main body 2 or may be separated from the main body 2. Alternatively, the display member 8 is not constituted by the main display 80 and the auxiliary display 81, but may be constituted by one display or three or more displays.

The ultrasonic diagnostic apparatus 1 may further include a moving unit 9. The moving unit 9 may connect the main body 2 and the display member 8. The moving unit 9 may be configured to allow the display member 8 to be moved from the main body 2. By means of the moving unit 9, the display member 8 may be disposed in a state of being spaced apart from the main body 2.

The main body 2 may be provided with the input 7. The input 7 may be provided in the form of a keyboard, a button, a dial, a foot switch, or a foot pedal. In a case where the input 7 is a keyboard, the input 7 may be provided at an upper portion of the main body 2. In a case where the input 7 is a foot switch or a foot pedal, the input 7 may be provided at a lower portion of the main body 2.

The input 7 may be provided by having a keyboard, a button, a dial, and the like on a panel 70. The panel 70 may be mounted to the main body 2. A handle 71 may be provided on one side of the panel 70. A user may move the ultrasonic diagnostic apparatus 1 by holding the handle 71 and applying a force.

The probe 3 may be connected to the main body 2 by a connection member 5. The connection member 5 includes a cable 50 and a connector 51. The probe 3 may be provided on one side of the cable 50 and the connector 51 may be provided on the other side of the cable 50. The connector 51 may be detachably mounted on a connection portion 20 provided on the main body 2. Accordingly, the probe 3 may be connected to the main body 2.

A rest portion 4 may be provided on one side of the ultrasonic diagnostic apparatus 1 so that the probe 3 may be rested in the main body 2. When the ultrasonic diagnostic apparatus 1 is not used, an inspector may put and store the probe 3 in the rest portion 4. As an example, the rest portion 4 may be provided on the panel 70 in the form of a hole in which a handle portion of the probe 3 may penetrate. The probe 3 may be rested in the panel 70 by being inserting into the hole formed on the panel 70. As another example, the rest portion 4 may be provided in the form of a holder mounted on the main body 2. The probe 3 may be inserted into and rested in the holder.

The main body 2 may be provided with a handle 21 and the handle 71 on a front side and a rear side thereof so that the user may move the ultrasonic diagnostic apparatus 1. The handles 21 and 71 may include the first handle 71 provided on the front side of the main body 2 and the second handle 21 provided on the rear side of the main body 2. The first handle 71 may be provided on one side of the panel 7. The second handle 21 may be provided to protrude from the rear of the main body 2.

The main body 2 may be provided with a plurality of casters 22 and 23 for moving the ultrasonic diagnostic apparatus 1. The casters 22 and 23 may be aligned so as to cause the main body 2 to travel in a specific direction (alignment movement mode), may be arranged so as to cause the main body 2 to freely move (free movement mode), or may be locked so as to cause the main body 2 to stop at a specific position (stop mode).

The casters 22 and 23 may include the first caster 22 and the second 23. When the direction in which the input 7 and the display member 8 are positioned is referred to as the front and the direction opposite thereto is referred to as the rear, the first caster 22 may be positioned on the front side of the main body 2, and the second casters 23 may be positioned on the rear side of the main body 2. The first casters 22 may be provided on both the left and right sides of the front side of the main body 2, respectively. The second casters 23 may be provided on both the left and right sides of the rear side of the main body 2, respectively, so as to correspond to the first casters 22.

The main body 2 may be provided with an operation member 25 capable of controlling the first and second casters 22. The operation member 25 may be provided in the form of a foot pedal as illustrated in FIG. 1, or may be provided in the form of a button, a dial, or the like. The user may grasp the first handle 71 and move or stop the ultrasonic diagnostic apparatus 1 after pressing and operating the foot pedal 25 with the foot.

Hereinafter a reference member according to an embodiment of the disclosure and an ultrasonic diagnostic apparatus capable of obtaining position information of a target object using the reference member will be described in detail.

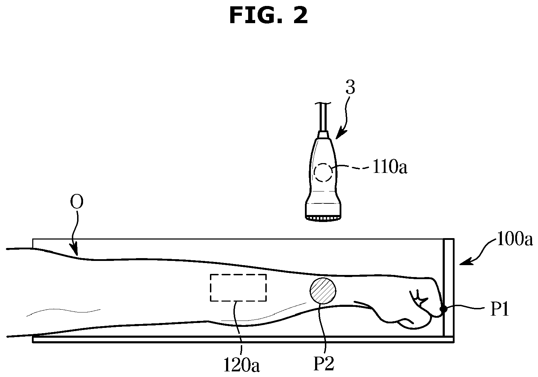

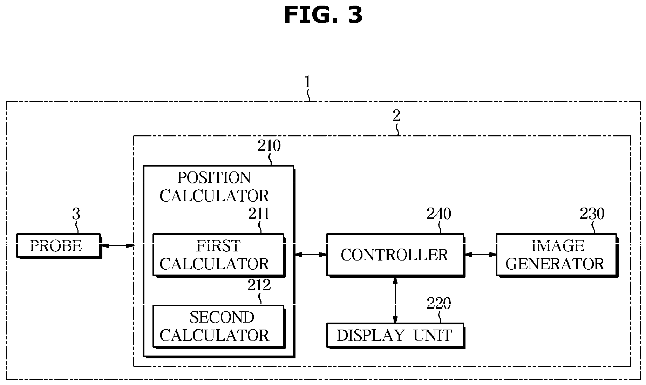

FIG. 2 is a view illustrating a reference member, a target object, and a probe in the ultrasonic diagnostic apparatus according to an embodiment of the disclosure, and FIG. 3 is a block diagram illustrating the configuration of the ultrasonic diagnostic apparatus according to an embodiment of the disclosure.

Referring to FIG. 2, the ultrasonic diagnostic apparatus 1 according to an embodiment of the disclosure may include a reference member 100a provided to set a reference position P1 in a target object O by making contact with a part of the target object O. The target object may include a person or animal, or a part of a person or animal. Hereinafter the case where the target object is a person will be described as an example.

The reference member 100a may be provided to receive a part of the arm of the target object O. For example, the reference member 100a may be provided in a rectangular parallelepiped shape with its upper surface opened.

The reference member 100a may include a reference setting part (not indicated by a reference numeral). The reference setting part may indicate one region of the reference member 100a that is provided to be in contact with the target object O. Referring to FIG. 2, the reference setting part may indicate a surface in contact with the target object O, that is, a surface including the reference position P1.

The reference position P1 of the target object O may be set by bringing a part of the target object O into contact with the reference setting part. The reference position P1 may indicate one point of the target object O in contact with the reference setting part. In FIG. 2, P1 may indicate one point of the target object O and at the same time indicate one point of the reference member 100a.

The reference member 100a may be provided such that at least a portion of the reference member 100a is in contact with the target object O. In this case, the at least a portion of the target object O in contact with the reference member 100a may be set as the reference position P1. The reference position, which is one point of the target object O, means a position serving as a reference point for displaying the position information of the target object. Also, the reference position may indicate one point of the reference member 100a. That is, the reference position may be one point of the target object O, and at the same time one point of the reference member 100a. In other words, the reference position may be a point where the target object O and the reference member 100a are in contact with each other.

The reference member 100a, which is a structure provided to set the reference position P1 in the target object O, is not limited in shape, size, number, and the like. The reference member 100a may cause the target object O to repeatedly assume the same posture. That is, in order for the target object O to be in contact with one point of the reference member 100a, it is necessary to assume a predetermined posture, and through this, the reference member 100a may obtain the same reference position P1 on the same target object O regardless of points of time.

Referring to FIG. 2, when the target object O stretches an arm horizontally with a fist held, one point (the reference position P1) of a finger may make contact with the reference member 100a. In this case, the one point of the finger may become the reference position P1. The reference position is not an unchanging position, and it may be changed at any time depending on the setting. Any point on the target object O may be a reference position as long as the target object O may assume the same posture and the same point in the target object O may be in contact with the reference member 100a.

The reason for setting the reference position is that a reference point of the target object O is required when it is necessary to continuously obtain ultrasonic images for the same site within the target object O at different points of time. Therefore, as long as the target object O may assume the same posture, the reference position may be changed at any time. Unlike in FIG. 2, the reference position is not set at one point of the finger, and one point of the palm may be set as the reference position. In this case, a mark indicating the reference position may be provided on the reference member 100a so that the same posture of the target object O may be secured. For example, in FIG. 2, a schematic picture of a fist may be provided on one surface of the reference member 100a facing the fist of the target object O. The target object O may assume a posture so that the fist of the target object O faces the fist picture provided on the reference member 100a. If there is no abrupt change in the body structure of the target object O, the same reference position may be set by assuming the same posture.

The ultrasonic image may indicate an image for one region inside the target object O at an arbitrary position P2, which will be described later. The arbitrary position P2 may indicate a coordinate in a three-dimensional space, and the ultrasonic image may mean an image inside the target object O including an arbitrary position P2.

The probe 3 may be configured to obtain ultrasonic data for the arbitrary position P2 inside the target object O. The arbitrary position P2 means one region inside the target object O to which the ultrasonic signal generated by a transducer (not shown) of the probe 3 is reflected. The arbitrary position P2 may indicate one point of an ultrasonic image. Therefore, the arbitrary position P2 may vary depending on the position of the probe 3 and the direction in which the probe 3 directs. As illustrated in FIG. 2, the arbitrary position P2 may be one point of the arm of the target object O.

According to an embodiment of the disclosure, the ultrasonic diagnostic apparatus 1 may include a marker 120a disposed to be spaced apart from the reference member 100a by a predetermined distance, and a sensor 110a provided to obtain a positional relationship with the marker 120a.

The marker 120a may be fixed at a predetermined position. The marker 120a may be disposed to have a predetermined positional relationship with the reference member 100a. The marker 120a and the reference member 100a may be disposed in one structure or may be disposed in different structures, respectively.

The sensor 110a may be provided to sense the position of the marker 120a. The sensor 110a may be disposed on one side of the probe 3. The sensor 110a may be provided to move with the probe 3. The sensor 110a may detect the energy generated from the marker 120a. The sensor 110a may detect the energy generated from the marker 120a to track the position of the marker 120a. That is, the sensor 110a may obtain a positional relationship with the marker 120a. In this case, the positional relationship may mean 3D coordinates of the sensor 110a with respect to the marker 120a.

Referring to FIG. 3, the ultrasonic diagnostic apparatus 1 may include a position calculator 210 for calculating a positional relationship between the reference position P1 and the arbitrary position P2 using the positional relationship between the marker 120a obtained by the sensor 110a and the sensor 110a, and a display unit 220 for displaying an ultrasonic image and the positional relationship.

The position calculator 210 may calculate the positional relationship between the reference position P1 and the arbitrary position P2. In this case, the positional relationship between the reference position P1 and the arbitrary position P2 may mean 3D coordinates of the arbitrary position P2 with respect to the reference position P1.

The position calculator 210 may include a first position calculator 211 for calculating a positional relationship between the reference position P1 and the marker 120a, and a second position calculator 212 for calculating a positional relationship between the sensor 110a and the arbitrary position P2. The positional relationship between the reference position P1 and the marker 120a may mean 3D coordinates of the marker 120a with respect to the reference position P1. The positional relationship between the sensor 110a and the arbitrary position P2 may mean 3D coordinates of the arbitrary position P2 with respect to the sensor 110a.

The first position calculator 211 may obtain the positional relationship between the reference position P1 and the marker 120a. As described above, the position of the marker 120a does not change because the marker 120a is fixed at the predetermined position. Also, the reference position P1 indicates one point of the reference member 100a, and its position does not change. Accordingly, the first position calculator 211 may obtain the positional relationship between the reference position P1 and the marker 120a through a predetermined calculation regarding a physical positional relationship between the reference position P1 and the marker 120a. Also, this positional relationship is constant irrespective of the movement of the probe 3 and/or the change of the target object O (when the patient is changed to another person). That is, the positional relationship between the reference position P1 and the marker 120a may have a fixed value.

As described above, the sensor 110a may detect the energy generated from the marker 120a to track the position of the marker 120a, and the first position calculator 211 may obtain the positional relationship between the reference position P1 and the marker 120a through calculation. Therefore, the positional relationship of the sensor 110a provided on the probe 3 from the reference position P1 may be obtained.

The second position calculator 212 may obtain the positional relationship between the sensor 110a and the arbitrary position P2.

It is necessary to preferentially obtain the positional relationship between the sensor 110a and the transducer (not shown) of the probe 3 in order to obtain the positional relationship between the sensor 110a and the arbitrary position P2.

Because the sensor 110a is fixed to one side of the probe 3 and the transducer is also fixed to the probe 3, the positional relationship between the sensor 110a and the transducer may have a fixed value. In the same way as the first position calculator 211 obtains the positional relationship between the reference position P1 and the marker 120a, the second position calculator 212 may obtain the positional relationship between the sensor 110a and the transducer through a predetermined calculation.

Hereinafter the process of obtaining the positional relationship between the probe 3 and the ultrasonic image at the arbitrary position P2 will be described.

The main body 2 may include a transmission signal generator (not shown). The transmission signal generator forms a transmission signal to be applied to each of the conversion elements of the probe 3 in consideration of the position and focusing point of a plurality of conversion elements (not shown) included in the probe 3. The transmission signal is a transmission signal for obtaining a frame of an ultrasonic image.

The probe 3 converts the transmission signal provided from the transmission signal generator into an ultrasonic signal and transmits the ultrasonic signal to the target object O. The probe 3 transmits an ultrasonic beam formed through the plurality of conversion elements and made up of a group of ultrasonic signals to a diagnosis site of the target object O. The probe 3 receives an ultrasonic echo signal reflected from the diagnosis site of the target object O and forms an electrical reception signal therefrom.

A beam former (not shown) converts an analog electrical reception signal provided from the probe 3 into a digital signal. The beam former applies a delay to the digitally converted electrical reception signal in consideration of the position and focusing point of the conversion element. Also, the beam former combines the delayed electrical reception signals to form a reception focusing beam.

By the electrical reception signals, the second position calculator 212 may obtain the positional relationship between the ultrasonic image and the transducer at the arbitrary position P2.

As described above, the positional relationship between the sensor 110a and the transducer may be calculated, and the positional relationship between the ultrasonic image at the arbitrary position P2 and the transducer may be obtained. Through this process, the positional relationship between the ultrasonic image at the arbitrary position P2 and the sensor 110a may be obtained. Also, the positional relationship between the sensor 110a and the arbitrary position P2 may be obtained.

Because the arbitrary position P2 varies depending on the position and/or the direction of the probe 3, the positional relationship between the sensor 110a and the arbitrary position P2 may have a variable value instead of a fixed value.

When the first position calculator 211 obtains the positional relationship between the reference position P1 and the marker 120a and the second position calculator 212 obtains the positional relationship between the sensor 110a and the arbitrary position P2, the positional relationship between the reference position P1 and the arbitrary position P2 may also be obtained. This is because the positional relationship between the marker 120a and the sensor 110a that the first position calculator 211 and the second position calculator 212 may not obtain is obtained by the sensor 110a as described above. That is, the positional relationship between the reference position P1 and the marker 120a may be obtained by the first position calculator 211, the positional relationship between the marker 120a and the sensor 110a may be obtained by the sensor 110a, and the positional relationship between the sensor 110a and the arbitrary position P2 may be obtained by the second position calculator 212, so that the positional relationship between the reference position P1 and the arbitrary position P2 may be obtained by taking the above positional relationships into consideration.

According to an embodiment of the disclosure, the ultrasonic diagnostic apparatus 1 may include an image generator 230 for generating an ultrasonic image using the ultrasonic data obtained by the probe 3, and the display unit 220 for displaying the ultrasonic image and the positional relationship obtained by the position calculator 210.

The probe 3 may obtain ultrasonic data for the arbitrary position P2 inside the target object O. The image generator 230 may generate an ultrasonic image for the arbitrary position P2 using the ultrasonic data obtained by the probe 3.

The display unit 220 may display the ultrasonic image generated by the image generator 230 and the positional relationship obtained by the position calculator 210 simultaneously or individually. The display unit 220 will be described in detail later.

The ultrasonic diagnostic apparatus 1 may include a controller 240 for determining whether or not a first position of the probe 3 at a first point of time and a second position of the probe 3 at a second point of time correspond to each other when the probe 3 obtains the ultrasonic data for the target object O at the first point of time and the second point of time different from the first point of time.

As the reference member 100a is provided, the target object O may assume the same posture regardless of points of time. Accordingly, the target object O and the reference member 100a may have the same reference position P1 regardless of points of time. The reference position P1 may have fixed coordinates if the target object O is the same (if the person is the same).

The arbitrary position P2 varies depending on the position and/or the direction of the probe 3. A user (for example, a doctor, a nurse, a medical laboratory technologist, etc.) of the ultrasonic diagnostic apparatus 1 may move the probe 3 in order to obtain an ultrasonic image of a predetermined body part (e.g., liver, kidney, heart, etc.) of the target object O. The predetermined body part of the target object may be different depending on the target object (for example, the patient). Also, even if the predetermined body part is the same because the target object is the same, it is not simple to obtaining an ultrasonic image of the same body part for the same target object. That is, in a case of a patient who has to observe the progress of treatment with time difference, an ultrasonic image at the same or similar 3D coordinates is required even for the same body part. This is because it is necessary to compare ultrasonic images at the same or similar coordinates to make accurate comparison.

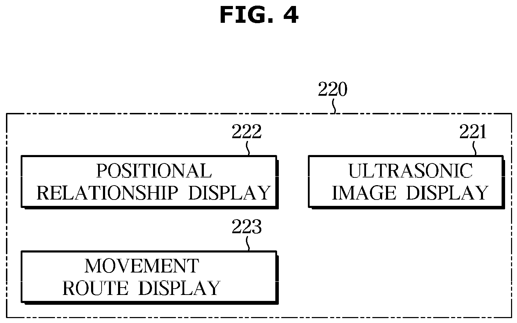

FIG. 4 is a block diagram illustrating the configuration of a display unit in the ultrasonic diagnostic apparatus according to an embodiment of the disclosure.

According to the disclosure, as described above, the ultrasonic diagnostic apparatus 1 may obtain the positional relationship between the reference position P1 and the arbitrary position P2, and this positional relationship may be displayed on the display unit 220. The user may obtain an ultrasonic image at the same 3D coordinates for the same target object with reference to the positional relationship.

The controller 240 may determine whether or not the position (first position) of the probe 3 at the first point of time and the position (second position) of the probe 3 at the second point of time correspond to each other.

When the first position and the second position correspond to each other, a predetermined operation may be performed. For example, the ultrasonic diagnostic apparatus 1 may inform the user that the first position and the second position of the probe 3 correspond to each other by a predetermined method. As an example of the predetermined method, the display unit 220 may display a predetermined indication that the first position coincides with the second position.

The controller 240 may select a movement route through which the probe 3 positioned at the second position moves to the first position when it is determined that the first position and the second position do not correspond to each other. In addition, the display unit 220 may display the movement route from the second position to the first position. When the user moves the probe 3 located at the second position to the first position with reference to the movement route, the controller 240 may inform the user that the first position and the second position of the probe 3 correspond to each other by the predetermined method.

Referring to FIG. 4, the display unit 220 may include an ultrasonic image display 221 for displaying the ultrasonic image generated by the image generator 230, a positional relationship display 222 for displaying the positional relationship obtained by the position calculator 210, and a movement route display 223 for displaying the movement route of the probe 3.

The ultrasonic image display 221, the positional relationship display 222 and the movement route display 223 may be simultaneously displayed on the display unit 220 or displayed on the display unit 220 individually or in partial combination.

When the first position and the second position of the probe 3 correspond to each other, the display unit 220 may display the ultrasonic image obtained at the first point of time and the ultrasonic image obtained at the second point of time. In this case, the ultrasonic image obtained at the first point of time and the ultrasonic image obtained at the second point of time may be displayed on the ultrasonic image display 221. Alternatively, the ultrasonic image obtained at the first point of time may be displayed on the ultrasonic image display 221, and the ultrasonic image obtained at the second point of time may be displayed on the positional relationship display 222 and/or the movement route display 223. In addition, the ultrasonic image may be displayed on the display unit 220 by various methods.

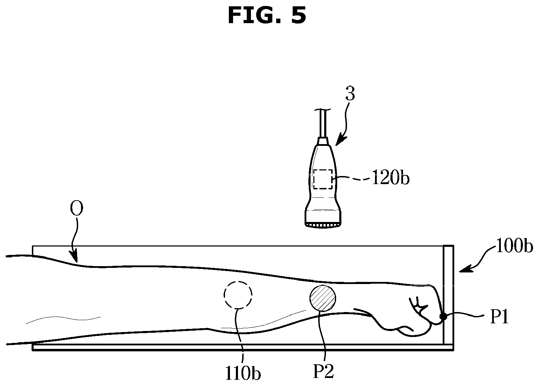

FIG. 5 is a view illustrating a reference member, a target object, and a probe in an ultrasonic diagnostic apparatus according to another embodiment of the disclosure.

Referring to FIG. 5, in an ultrasonic diagnostic apparatus according to another embodiment of the disclosure, the probe 3 may include a marker 120b. A sensor 110b may be disposed to be spaced apart from a reference member 100b by a predetermined distance. The sensor 110b may be fixed at a predetermined position and the marker 120b may move together with the probe 3.

The sensor 110b in a fixed state may detect the position of the marker 120b. The structure and function of the remaining components except that the sensor 110b is fixed at a predetermined position and the marker 120b is provided to be movable together with the probe 3 is the same as those of the ultrasonic diagnostic apparatus 1 illustrated in FIGS. 1 to 4, and thus duplicate description will be omitted.

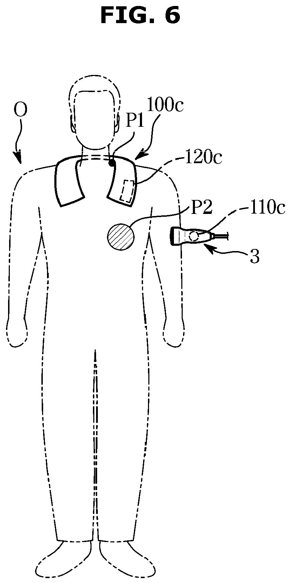

FIG. 6 is a view illustrating a reference member, a target object, and a probe in an ultrasonic diagnostic apparatus according to another embodiment of the disclosure.

Referring to FIG. 6, in an ultrasonic diagnostic apparatus according to another embodiment of the disclosure, a reference member 100c may be provided substantially in the shape of a neck pillow. The reference member 100c may include a marker 120c disposed on one side thereof. The probe 3 may include a sensor 110c.

The reference member 100c may be provided to cover the neck and an upper portion of the chest of the target object O. The target object O may have the same reference position P1 by wearing the reference member 100c.

As illustrated in FIG. 6, the reference member 100c and the target object O do not make contact at any one point but may make surface contact. The reference member 100c may be provided to surround the neck, the shoulder, and a part of the chest of the target object O. The reference position P1 indicates one point at which the target object O and the reference member 100c are in contact with each other, and the reference position P1 may be set as any one point on the reference member 100c because the target object O and the reference member 100c are in surface contact. Although not illustrated in the drawing, because the marker 120c is disposed on one side of the reference member 100c, the positions of the reference position P1 and the marker 120c may coincide with each other.

When the positions of the reference position P1 and the marker 120c coincide with each other, the position calculator 210 may not include the first position calculator 211. That is, the first position calculator 211 may be omitted. This is because the positions of the reference position P1 and the marker 120c coincide with each other and thus the positional relationship between the reference position P1 and the marker 120c need not be obtained.

FIG. 7 is a view illustrating a reference member, a target object, and a probe in an ultrasonic diagnostic apparatus according to another embodiment of the disclosure.

Referring to FIG. 7, in an ultrasonic diagnostic apparatus according to another embodiment of the disclosure, a reference member 100d may be provided in a structure similar to a belt. The reference member 100d may include a marker 120d disposed on one side thereof. The probe 3 may include a sensor 110d.

The reference member 100d may be provided to surround the abdomen of the target object O. The target object O may have the same reference position P1 by wearing the reference member 100d.

As illustrated in FIG. 7, the reference member 100d and the target object O do not make contact at any one point but may make surface contact. The reference position P1 indicates one point at which the target object O and the reference member 100d are in contact with each other, and the reference position P1 may be set as any one point on the reference member 100d because the target object O and the reference member 100d are in surface contact. Although not illustrated in the drawing, because the marker 120d is disposed on one side of the reference member 100d, the positions of the reference position P1 and the marker 120d may coincide with each other.

When the positions of the reference position P1 and the marker 120d coincide with each other, the position calculator 210 may not include the first position calculator 211. That is, the first position calculator 211 may be omitted. This is because the positions of the reference position P1 and the marker 120d coincide with each other and thus the positional relationship between the reference position P1 and the marker 120d need not be obtained.

As is apparent from the above, according to an aspect of the disclosure, an ultrasonic diagnostic apparatus capable of easily obtaining an ultrasonic image for the same position of the same target object at different points of time can be provided.

According to another aspect of the disclosure, an ultrasonic diagnostic apparatus capable of setting a reference position to a target object using a reference member and identifying a position of a probe with respect to the reference position can be provided.

While the present disclosure has been particularly described with reference to exemplary embodiments, it should be understood by those of skilled in the art that various changes in form and details may be made without departing from the spirit and scope of the present disclosure.

## Claims

1. An ultrasonic diagnostic apparatus comprising:
a probe configured to obtain ultrasonic data for an arbitrary position inside a target object;
an image generator to generate an ultrasonic image for the arbitrary position using the ultrasonic data;
a reference member provided to be in contact with the target object to set a reference position in the target object;
a marker disposed on one side of the reference member;
a sensor disposed on one side of the probe and configured to obtain a positional relationship with the marker; and
a position calculator configured to calculate a positional relationship between the reference position and the arbitrary position using the positional relationship between the marker and the sensor.

2. The ultrasonic diagnostic apparatus according to claim 1, further comprising
a display to display the ultrasonic image and the positional relationship.

3. The ultrasonic diagnostic apparatus according to claim 1 or 2, wherein
the reference member includes a reference setting part provided to be in contact with the target object, and the reference member sets one point of the target object in contact with the reference setting part as the reference position.

4. The ultrasonic diagnostic apparatus according to any of the previous claims, wherein
the position calculator includes
a first position calculator to obtain a positional relationship between the reference position and the marker, and a second position calculator to obtain a positional relationship between the sensor and the arbitrary position.

5. The ultrasonic diagnostic apparatus according to any of the previous claims in as far as depending on claim 2, wherein
when the probe obtains ultrasonic data for the target object at a first point of time and a second point of time different from the first point of time, the display displays an ultrasonic image for a first position obtained at the first point of time and an ultrasonic image for the first position obtained at the second point of time.

6. The ultrasonic diagnostic apparatus according to any of the previous claims in as far as depending on claim 2, further comprising
a controller, when the probe obtains ultrasonic data for the target object at a first point of time and a second point of time different from the first point of time, to determine whether or not a first position of the probe at the first point of time and a second position of the probe at the second point of time correspond to each other.

7. The ultrasonic diagnostic apparatus according to claim 6, wherein
the display, when the first position and the second position correspond to each other, displays an ultrasonic image for the first position obtained at the first point of time and an ultrasonic image for the second position obtained at the second point of time.

8. The ultrasonic diagnostic apparatus according to any of the previous claims in as far as depending on claim 2, further comprising
a controller, when the probe obtains ultrasonic data for a second position of the target object at a second point of time different from a first point of time after obtaining ultrasonic data for a first position of the target object at the first point of time, to determine whether or not the first position and the second position correspond to each other.

9. The ultrasonic diagnostic apparatus according to claim 8, wherein
the controller, when it is determined that the first position and the second position do not correspond to each other, selects a movement route for the probe to move to the first position, and the display displays the movement route from the position of the probe to the first position.
